# EUROPEAN PATENT APPLICATION

(11) **EP 4 382 027 A1**
(43) Date of publication of application: **12.06.2024**
(21) Application number: 22211500.8
(22) Date of filing: 05.12.2022
(51) Int. Cl.: A61B 3/00, A61B 3/028, A61B 3/06, A61B 3/10, A61B 3/107, A61B 3/11

(54) **DETERMINING AT LEAST ONE VALUE FOR AN OCULAR ABERRATION**

(71) Applicant: Carl Zeiss Vision International GmbH, 73430 Aalen (DE)
(72) Inventor: Buehren, Tobias, 89073 Ulm (DE); Delbert, Andrews, 73447 Oberkochen (DE); Trost, Michael, 07646 Stadtroda (DE); Volkwardt, Martin, 07646 Stadtroda (DE)
(74) Representative: Altmann Stößel Dick Patentanwälte PartG mbB

(57) **Abstract**

The present invention relates to a method, an apparatus, and a computer program product for determining at least one value (328) for an ocular aberration of at least one eye (112) of a user (114) as well as to a related method for producing at least one spectacle lens for the at least one eye of the user. Herein, the method comprises the steps:
a) providing input data (314), the input data (314) comprising:
- first information (118) about an ocular aberration of at least one eye (112) of a user (114) at a point in time (316);
- second information (126) about at least one geometric and optical property of the at least one eye (112) of the user (114) at the same point in time (316); and
- third information (128) about at least one geometric and optical property of the at least one eye (112) of the user (113) at a previous point in time (320),
and

b) generating output data (326), the output data (326) comprising the at least one value (328) for the ocular aberration of the at least one eye (112) of the user (114), wherein the output data (326) is generated by
- comparing the second information (126) with the third information (128); and
- adjusting the first information (118),

wherein the second information (126) and the third information (128) is represented in a model (210) of the at least one eye (112) of the user (114), the model (210) of the at least one eye (112) of the user (114) being based on a value for each of:
- an axial length (224);
- a front curvature (226), a back curvature (228), a depth (230), and at least one refractive index of a cornea (212);
- a depth (232) of an anterior chamber (216) and at least one refractive index of an aqueous humor (233) in the anterior chamber (216);
- a front curvature (234), a back curvature (236), a depth (238), and at least one refractive index of a crystalline lens (214); and
- a depth (242) and at least one refractive index of a vitreous chamber (219) of the at least one eye (112) of the user (114), wherein each geometric value is provided with respect to an axis (222) on which a reference coordinate system (244) is based.

## Description

### Field of the invention

The present invention relates to a method, an apparatus, and a computer program product for determining at least one value for an ocular aberration of at least one eye of a user as well as to a related method for producing at least one spectacle lens for the at least one eye of the user.

### Related art

Methods and devices for determining at least one ocular aberration of at least one eye of a user are known. As generally used, the term "ocular aberration" refers to a difference between a surface of an ideal optical wavefront and a surface of an actual optical wavefront which is determined for the at least one eye of a user. Herein, the term "optical wavefront" relates to a surface which is perpendicular to a beam along which light propagates. Within a typical human population, the ocular aberration, usually, comprises at least one second-order spherocylindrical focus error, also denoted as "refractive error", wherein, however, at least one higher-order aberration may also occur.

US 2014/0176904 A1 discloses an ophthalmic aberrometer combining measurements of wavefront aberrations and subjective refraction into a single instrument and refers both measurements to the same corneal plane. It further discloses an ophthalmic aberrometer employing an open field and subjective correction to overcome instrument myopia and to ensure accurate measurement of the best-corrected visual acuity in addition to measurement of wavefront aberrations. It further discloses an ophthalmic aberrometer implementing an optical relay with adjustable optical power compensation to eliminate the need for flipping plurality sets of trial lenses for defocus correction. It further discloses an ophthalmic aberrometer making wavefront measurement along a viewing path of the subject eye and enabling accurate measurement of the residual wavefront aberrations after compensating for the subjective refraction.

US 2020/0229691 A1 discloses a method for testing the eyes of a test person with the aid of a vision testing system as well as vision testing system, comprising a first measuring device, a second topographic measuring device, a third refractive measuring device and a processing means, an axial length (L) of an eye of the test person being measured with the aid of the first measuring device, a curvature of the cornea of the eye being measured with the aid of the second measuring device, a refractive property of the eye being measured with the aid of the third measuring device, a measurement simultaneously being carried out with the first, second and third measuring device at the eye, measurement data of the measurements of the first, second and third measuring device being processed with the aid of the processing means, said processing means presenting a database with normal data, a comparison of the measurement data with the normal data being carried out and a result of said comparison being issued by means of said processing means.

Despite the advantages of existing methods and devices for determining at least one values for an ocular aberration of at least one eye of a user, there is still room for improvement. In particular, non-cycloplegic auto-refraction is known to be inaccurate and not suitable for studies of refractive error. Population-based studies on the prevalence of refractive error in school-children have encountered difficulties since numerous parents and children refuse to undertake cycloplegic refraction owing to blurred vision and photophobia after cycloplegia. In general, non-cycloplegic auto-refraction overestimates myopia and underestimates hyperopia in children whose accommodative responses are active. Therefore, a need exists for achieving reliable non-cycloplegic auto-refraction results, especially for population-based studies on the refractive error in children but also for accurate refractive error progression monitoring in myopia progression management in children.

### Problem to be solved

In particular with respect to the disclosure of US 2020/0229691 A1, it is an objective of the present invention to provide a method, a device, and a computer program product for determining at least one value for an ocular aberration of at least one eye of a user as well as to a related method for producing at least one spectacle lens for the at least one eye of the user, which at least partially overcome the above-mentioned problems of the state of the art.

It is a particular objective of the present invention to provide methods and devices for determining values for the ocular aberration of one or both eyes of the user having a high reliability, accuracy and repeatability compared to existing methods and devices. Herein, it would be desirable to be able to determine values for the ocular aberration having an accuracy of less than 0.25 dpt. It would, especially, be desirable to be able to determine values not only for second-order ocular aberrations, such as for the refraction, but also for higher-order ocular aberrations with a high reliability, accuracy and repeatability.

### Summary of the invention

This problem is solved by a method, an apparatus, and a computer program product for determining at least one value for an ocular aberration of at least one eye of a user as well as to a related method for producing at least one spectacle lens for the at least one eye of the user having the features of the independent claims. Preferred embodiments, which can be implemented in an isolated fashion or in any arbitrary combination, are listed in the dependent claims and throughout the description.

In a first aspect, the present invention relates to a method for determining at least one value for an ocular aberration of at least one eye of a user. Instead of the term "user", a different term, such as "subject", "person", "test person" or "wearer of eye glasses", may also be applicable. As already indicated above, the term "ocular aberration" refers to a difference between a surface of an ideal optical wavefront and a surface of an actual optical wavefront which is determined for the at least one eye of a user. As further already defined above, the term "optical wavefront" relates to a surface which is perpendicular to a beam along which light propagates. Further, the term "light" refers to electromagnetic radiation in at least one of the visible spectral range or the infrared spectral range. As generally used, the term "visible spectral range" refers to electromagnetic radiation having a wavelength of 380 nm to 780 nm, whereas the term "infrared spectral range" relates to electromagnetic radiation having a wavelength above 780 nm to 1000 µm, wherein the visible spectral range or the "near infrared spectral range", which refers to electromagnetic radiation having a wavelength above 780 nm to 1.5 µm, may particularly be preferred. Further, the term "light beam" relates to a propagation of the light in form of at least one ray, wherein a direction of propagation of the at least one ray is, generally, denoted by the term "optical path", wherein the optical path may be modified by at least one optical element, particularly selected from a mirror, a beam splitter, or a diffractive element, such as an optical grating.

In general, the method according to the present invention can be performed in a manual fashion in which a trained eye care professional may perform the indicated steps by using an appropriate device, preferably the apparatus for determining at least one value for an ocular aberration of at least one eye of a user as disclosed elsewhere herein. However, in a particularly preferred embodiment, the method according to the present invention may be a computer-implemented method. As generally used, the term "computer-implemented method" refers to a method that involves at least one device, specifically a computer, or a plurality of devices, particularly connected via a computer network. The plurality of devices may be connected, particularly for transmitting data, via a network by using at least one connection interface at any one of the devices of the plurality of devices. Alternatively or in addition, at least one device may be a mobile communication device, wherein the term "mobile communication device" refers to a portable wireless telecommunication equipment, especially a smartphone, a notebook, a personal digital assistant, or a laptop, which is configured to transmit and/or receive computer data, voice, or video. However, a further kind of device may be conceivable.

The computer-implemented method may be implemented as at least one computer program provided on a storage medium, especially a non-transient storage medium, carrying the computer program, whereby at least one of the steps of the computer-implemented method, specifically at least one of, preferably both, steps a) or b) of the present method, are performed by using the at least one computer program. Alternatively, the at least one computer program may be accessible by the device which may be adapted for performing the method via a network, such as via an in-house network, via internet, or via a access to a cloud. With particular regard to the present invention, the present method can be performed on a programmable device being configured to this purpose, such as by providing a computer program being is configured to such a purpose.

As generally used, the term "determining" or any grammatical variation thereof refers to a process that is configured to generate at least one representative result. The representative result may be determined in a process, wherein at least one step of the process may be selected from at least one of: a measurement step; an evaluation step; or a displaying step. For determining the representative result, at least one measurement may be performed. Data generated in this measurement may be evaluated. The representative result may be data retrieved in the evaluation process. The representative result may be displayed. With regard to the present invention, the representative result comprises output data comprising the at least one value for the ocular aberration of the at least one eye of the user. The outcome data may be displayed on at least one screen.

The method may be performed for exactly one eye of the person simultaneously. The method may be repeated, in a consecutive fashion, for a further eye of the person. The method may further be implemented for being performed for at least one eye of the person or both eyes of the person or any eye of the person simultaneously.

The method for determining at least one value for an ocular aberration of at least one eye of a user comprises the following steps a) and b), which may be performed in the given order. However, both method steps a) and b) may be performed simultaneously, wherein the method steps a) and b) may at least partly overlap in time. Further, one or both method steps a) and b) may be performed once or repeatedly. The method may comprise additional method steps, independently of whether they are disclosed herein or not.

The steps of the present method are as follows:
a) providing input data, the input data comprising:
   - first information about an ocular aberration of at least one eye of a user at a point in time;
   - second information about at least one geometric and optical property of the at least one eye of the user at the same point in time; and
   - third information about at least one geometric and optical property of the at least one eye of the user at a previous point in time,
   and
b) generating output data, the output data comprising the at least one value for the ocular aberration of the at least one eye of the user, wherein the output data is generated by
   - comparing the second information with the third information; and
   - adjusting the first information,
wherein the second information and the third information is represented in a model of the at least one eye of the user, the model of the at least one eye of the user being based on a value for each of:
- an axial length;
- a front curvature, a back curvature, a depth, and at least one refractive index of a cornea;
- a depth of an anterior chamber and at least one refractive index of an aqueous humor in the anterior chamber;
- a front curvature, a back curvature, a depth, and at least one refractive index of a crystalline lens; and
- a depth and at least one refractive index of a vitreous chamber
of the at least one eye of the user, wherein each geometric value is provided with respect to an axis on which a reference coordinate system is based.

The present method and apparatus for determining at least one value for an ocular aberration of at least one eye of a user can, preferably, be used in a method for producing at least one spectacle lens for the at least one eye of the user as described below in more detail. Based on standard ISO 13666:2019, also referred to herein as the "standard", Section 3.5.2, the term "spectacle lens" relates to an optical lens which is used within the framework of the present invention for determining at least one ocular aberration of at least one eye of a user, wherein the spectacle lens is carried in front of the eye of the user. Usually, the ocular aberration within a typical human population comprises at least one second-order spherocylindrical focus error, also denoted as "refractive error". For describing a spherocylindrical lens which is designed for correcting a spherocylindrical focus error, various approaches are possible.

As defined in the standard, Section 3.6.6, the term "spherocylindrical lens" refers to a spectacle lens having spherical power and cylindrical power. Further, the spherocylindrical lens is defined, according to Section 3.13.1, as a spectacle lens which combines a paraxial, parallel beam of light in two individual, mutually perpendicular focal lines, whereby the spectacle lens has an apex refractive power only in two meridians. Further, the term "vertex refractive power" is, according to Section 3.10.7, defined as a reciprocal value of the width of the paraxial section. As further defined in Section 3.2.12 and 3.13.2, the term "meridian" relates to one of two perpendicular planes of the spectacle lens having an astigmatic effect being parallel to the two focal lines. Herein, the term "astigmatic effect" corresponds to an "astigmatic difference" which is defined in Section 3.13.6 as a difference between the value of the apex refractive power in the second meridian and the value of the apex refractive power in the first meridian. Further, the "cylindrical power" refers, according to Section 3.13.7, to an algebraic difference between the refractive values of the meridians, wherein the refractive value of a particular meridian being used as a reference is subtracted from the refractive value of the other meridian, while the "cylinder axis" indicates according to Section 3.13.8 the direction of the meridian of the spectacle lens whose apex refractive index is used as the reference.

As an alternative, L. N. Thibos, W. Wheeler und D. Horner (1997), Power Vectors: An Application of Fourier Analysis to the Description and Statistical Analysis of Refractive Error, Optometry and Vision Science 74 (6), S. 367-375, propose to approach the description of a spherocylindrical lens from a viewpoint of Fourier analysis of a power profile. They show that the familiar sine-squared law leads naturally to a Fourier series representation with exactly three Fourier coefficients, representing natural parameters of a thin lens. Herein, a constant term corresponds to a mean spherical equivalent (MSE) power, whereas amplitude and phase of the harmonic correspond to the power and axis of a Jackson cross-cylinder (JCC) lens, respectively. Expressing the Fourier series in rectangular form leads to the representation of an arbitrary spherocylindrical lens as sum of a spherical lens and two cross-cylinders, one at axis 0° and the other at axis 45°. The power of these three component lenses may be interpreted as (x, y, z) coordinates of a vector representation of the power profile. The power vector representation of a spherocylindrical lens can be used for numerical and graphical analysis of optometric data for problems involving lens combinations, comparison of different lenses, and statistical distribution of refractive errors.

According to step a), input data is provided. As generally used, the term "input data" refers to a set or a sequence of data that comprises at least one piece of information which is at least partly evaluated by a method, especially a computer-implemented method, in particular the method for determining at least one value for an ocular aberration of at least one eye of a user according to the present invention. As further generally used, the term "providing" or any grammatical deviation thereof refers to a process which is configured to make available at least one piece of information to at least one method step, especially the input data for starting step a) of the method. As described below in more detail, the input data are made available for generating the output data by using step b) of the method.

In accordance with the present invention, the input data comprises the following pieces of information:
- first information about an ocular aberration of at least one eye of a user at a point in time;
- second information about at least one geometric and optical property of the at least one eye of the user at the same point in time; and
- third information about at least one geometric and optical property of the at least one eye of the user at a previous point in time.
Herein, the terms "first", "second" and "third", are considered as a description of an element without specifying an order or a chronological sequence and without excluding a possibility that other elements of the same may be present.

Accordingly, the first information comprises at least one piece of information that refers to a particular value that is related to an ocular aberration of at least one eye of a user and to a particular point in time. As generally used, the term "point in time" refers to a temporal value that is related to a moment at which a particular event occurs or is performed by a person, device or process, either only at this temporal value or during a time interval for which this temporal value provides a representative value. Owing to the observation that values related to an ocular aberration of at least one eye of a user do not rapidly change within a short time interval but remain constant for months or years, it can be sufficient to indicate the point in time used in relation to the first information as a particular minute, hour, day, or week.

In a preferred embodiment, the particular value related to the ocular aberration of the at least one eye of the user may be a measured value. As used herein, the term "measured value" indicates that the particular value related to the ocular aberration of the at least one eye of the user has been captured by actually performing a measurement at the corresponding eye of the user at the particular point in time by applying at least one first measuring device that is configured for this purpose, preferably be selected from at least one of an automated refractor or a wavefront sensing device.

As generally used, the terms "automated refractor" or "autorefractor" refer to a type of optical measuring device which is configured to determine a measured value for a second-order ocular aberration, in particular the refraction, of at least one eye of a user in an automatic manner. Herein, a computer-implemented process is used to perform an examination of the eye of the user in order to generate a measured value for the second-order ocular aberration, in particular the at least one refractive error, especially a value for a sphere, a cylinder and an axis, of the eye of the user. For this purpose, reflections from a cone of light having at least one wavelength in the near infrared range can be detected to determine size and shape of a ring on the retina. By altering a magnification until an image enters into the focus, it can be derived how the eye focuses the image. This process can repeated in at least three meridians of the eye to allow the autorefractor calculates the refraction of the eye. However, a further kind of process may also be feasible.

As further generally used, the term "wavefront sensing device" refers to a further type of optical measuring device which is designated for determining aberrations of an optical wavefront, wherein the term is, usually, applied to an optical measuring device that does not require interference with a reference beam having no aberrations. Herein, the wavefront sensing device may, preferably be selected from at least one of: a Shack Hartmann wavefront sensor, a camera designated for measuring at least one point-spread function of an eccentric wavefront, a circular lenslet array aberrometer, a pyramid wavefront sensor, a phase element based wavefront sensor, or a ray tracing aberrometer. However, a further kind of wavefront sensing device may also be feasible. In particular, the wavefront sensing device may, thus, be used for determining not only a second-order ocular aberration but also a higher-order ocular aberration, which refers to at least one of a third-order aberration or a higher than a third-order aberration of the at least one eye of the user.

As generally used, the term "Shack Hartmann wavefront sensor" refers to a particular type of wavefront sensing device which comprises an array of individual small lenses which are, usually, denoted by the term "lenslets" and a two-dimensional optical detector, such as a CCD array, a CMOS array, or a quad-cell, wherein, upon uniform illumination of the lenslets, an integrated gradient of the incident optical wavefront across each lenslet is proportional to a displacement produced by each individual lenslet. In other words, an aberration of a phase of the incident optical wavefront can, thus, be approximated by a set of local tilts corresponding to the individual lenslets, wherein the tilts corresponding to the lenslets can also denoted by the term "eccentricities". By sampling the incident optical wavefront by virtue of the array of the lenslets in this fashion, the incident optical wavefront can, thus, at least partially, preferably completely, be reconstructed by measuring the local eccentricity of each individual lenslet within the array of the lenslets.

Further, the second information comprises at least one further piece of information that is related to at least one geometric and optical property of the at least one eye of the user and to the same point in time. As generally used, the term "same point in time" refers to a temporal value which is related to a particular event that occurs or is performed by a person, device or process with respect to a further particular event, wherein both particular events occur during the same interval, especially concurrently or consecutively. Since, as indicated above, the values related to an ocular aberration of at least one eye of a user do not rapidly change within a short time interval but remain constant for months or years, it can be sufficient to determine the second information within the identical minute, hour, day, or week as the first information. In a preferred embodiment, the particular value related to the at least one geometric and optical property of the at least one eye of the user may be a measured value. Herein, both the at least one measured value related to the first information and the at least one measured value related to the second information may, preferably, be recorded by using a combined apparatus that may comprise the at least one first measuring device configured to generate the first information, at least one second measuring device configured to generate the second information and the third information as described below in more detail, and a processing device further configured to control both the at least one first measuring device and the at least one second measuring device. In this manner, it can be ensured that the second information can actually be determined within the same point in time as the first information, in particular in a concurrent fashion or in a consecutive manner.

In a preferred embodiment, the particular value related to the at least one geometric and optical property of the at least one eye of the user may be measured at the same point in time by applying at least one second measuring device that is configured for this purpose, preferably for applying an imaging method to at least one image of the at least one eye of the user. As generally used, the term "image" refers to a two-dimensional graphical representation of the at least one object or the portion thereof. As further generally used, the term "imaging method" refers to a process of generating the two-dimensional graphical representation of the at least one object or the portion thereof. Preferably, a scanning process may be applied which comprises recording a plurality of images, each covering a partition, preferably an adjacent partition, of the object or the portion thereof, thus, enabling generating a three-dimensional graphical representation of the at least one object or the portion thereof by using the imaging method. In particular, the at least one image may completely cover the at least one eye of the user or at least a portion thereof that includes all features to be used in the model of the at least one eye of the user as disclosed below in more detail.

In a particularly preferred embodiment, the at least one second measuring device may be an optical coherence tomograph which is configured to use optical coherence tomography as the imaging method. As generally used, the term "optical coherence tomography", also abbreviated "OCT", denotes an imaging method for generating a two-dimensional or a three-dimensional recording of biological tissue, in particular of the eye of the user or a portion thereof, wherein a resolution in a micrometer range can preferably be obtained. To generate the desired recording, light having a temporally short coherence length from a radiation source is split into two parts in a beam splitter, wherein a first part of the light impinges on the tissue along an optical axis, wherein a second part of the light is guided over a reference path, and wherein the light reflected by the tissue is made to interfere with the reference light guided over the reference path to generate an interference signal. From the interference signal generated in this fashion, structures in the tissue along the optical axis can initially be distinguished; however, the desired two- or three-dimensional recording of the tissue or a section thereof can be generated by way of a lateral scanning by altering the optical axis over the tissue.

Preferably, the optical coherence tomography method can be selected from a Fourier domain OCT or a time domain OCT, wherein the Fourier domain OCT, especially spectral domain OCT or swept source OCT, is particularly preferred. The term "time domain OCT" denotes a procedure in which a length of the reference path is altered and an intensity of the interference is continuously captured in the process, with a change in the frequency spectrum of the interference remaining unconsidered. In contrast hereto, "Fourier domain OCT" denotes a procedure in which the change in components of the frequency spectrum of the interference is taken into account. If the change in the frequency spectrum of the interference is simultaneously excited and captured by using a broadband radiation source, this procedure is referred to as "spectral domain OCT". In contrast hereto, in "swept source OCT", the components of the frequency spectrum are temporally successively excited and captured, in particular by successive tuning of the frequency excitation of the radiation source.

Further, the third information comprises at least one further piece of information that is, again, related to the at least one geometric and optical property of the at least one eye of the user, however, it relates to a previous point in time. As generally used, the term "previous point in time" refers to a further temporal value which precedes a particular temporal value, especially in manner that a further event that has occurred or been performed by a person, device or process at the previous point in time has happened earlier with respect to the particular event. Since, as indicated above, the values related to the ocular aberration of at least one eye of a user do not rapidly change within a short time interval but remain constant for months or years, it can be sufficient to use the third information which relates to a previous point in time that has passed at least one year or at least two years but not more than five years.

In a preferred embodiment, the particular value related to the at least one geometric and optical property of the at least one eye of the user may be a measured value which has been recorded at least one year or at least two years but not more than five years ago. Herein, the at least one measured value related to the third information may, preferably, be recorded by using the same device or, preferably, the same type of device that has been used for recording the second third information. As generally used, the term "same type" refers to a class of devices which operate on the same underlying principle. In this manner, it can be ensured that the second information can reliably be compared with the third information, although the respective measurement may be performed on different sites. By way of example, whereas the third information may have been acquired at the previous point in time under supervision of an ophthalmologist, the second information and also the first information can be acquired later under supervision of an optometrist or an optician. In general, the at least one second measuring device which is configured to generate the second information is also configured to generate the third information. As already disclosed above, the at least one second measuring device is configured for applying an imaging method to at least one image of the at least one eye of the user, irrespective at which point in time the at least one image of the at least one eye of the user is recorded. For this purpose, the at least one second measuring device may be an optical coherence tomograph as described above in more detail which is configured to use optical coherence tomography as the imaging method.

In accordance with the present invention, the second information and the third information are represented in a model of the at least one eye of the user, particularly denoted by one of the terms "eye model" or "eye twin". As generally used, the term "model" refers to a simulation which is configured to provide a geometric and optical representation at least of essential features of the eye of the user, especially for an event in which the at least one eye of the user is involved in a fixation of a target, in a numerical fashion to be processed by using a computer. As further used herein, the term "essential features" refers to a portion of the eye of the user which covers all parts of the eye of the user that are involved in generating the image on the retina of the eye of the user. As further generally used, the term "is represented in" refers to an order and/or a form of presentation of data. Herein, the second information and the third information are provided by using the geometric and optical representation of at least the essential features of the eye in the particular eye model. In particular, the features of the eye which are determined by capturing the third information may, preferably, be used, to establish the particular model of the at least one eye of the user for later use, especially for comparing the second information with the third information. As generally used, the term "establishing" or any grammatical deviation thereof refers to a process of assigning at least one value to each feature of the model by using particular data, herein the values which comprised by the third piece of information. In this manner, the simulation may be configured to provide the geometric and optical representation of the visual process occurring within the eye of the user as complete as reasonable, thereby contributing to an increased reliability, accuracy and repeatability of the model of the at least one eye of the user compared to existing approaches, such as in US 2020/0229691 A1.

In a particularly preferred embodiment, fourth information about an ocular aberration of at least one eye of a user that may have been recorded at the same previous point in time at which the third information about the at least one geometric and optical property of the at least one eye of the user has been recorded, may be used for performing a calibration process configured for adapting at least one numerical parameter of the model of the at least one eye of the user. For this purpose, the at least one numerical parameter of the model of the at least one eye of the user as disclosed elsewhere herein in more detail may be adapted to at least one value comprised by the fourth information, in particular by employing an adaption process, especially by using a fitting procedure or a machine learning algorithm. However, a further manner of adaption of the at least one numerical parameter of the model of the at least one eye of the user to the at least one value comprised by the first information may also be conceivable. For the calibration, not only values for lower-order ocular aberrations, such as second-order aberrations, but also for at least one higher-order ocular aberration may, advantageously, be used, which may, especially, allow further increasing the reliability, accuracy and repeatability of the determination of the ocular aberration.

As further generally used, the term "numerical fashion" refers to a kind of representation of at least one feature by attributing at least one numerical parameter to each feature. The numerical parameters that are related to the essential features of the eye of the user, which are represented by the model of the at least one eye of the user according to the present invention, comprise at least one value for each of:
- an axial length;
- a front curvature, a back curvature, a depth, and at least one refractive index of a cornea;
- a depth of an anterior chamber and at least one refractive index of an aqueous humor in the anterior chamber;
- a front curvature, a back curvature, a depth, and at least one refractive index of a crystalline lens; and
- a depth and at least one refractive index of a vitreous chamber
of the at least one eye of the user. Herein, each of these features collectively contribute to the geometric and optical representation of the visual process by exerting a particular influence on the incident light propagating along the axis within the eye of the user.

Herein, each geometric value is provided with respect to an axis on which a reference coordinate system is based. As used herein, the term "geometric value" is a kind of value for a geometric extension, in particular a length, a width, or a depth. In contrast hereto, a value for the at least one refractive index of a particular substance in the at least one eye of the user which cannot be provided with respect to an axis in a coordinate system can, therefore, not be considered as being a geometric value. As generally used, the term "coordinate system" provides a definite reference for each spatial location in two or three spatial dimensions to be used by the model. As further generally used, the term "spatial location" indicates a position of each feature within a coordinate system. Preferably, the coordinate system to be used by the model of the at least one eye of the user according to the present invention may be a cylindrical coordinate system. As generally used, the "cylindrical coordinate system" is defined by a direction of an axis in space, a particular a location along the axis and a distance from the particular location in one or two dimensions along the axis. In this manner the reference coordinate system is based on the axis. The cylindrical coordinate system is particularly preferred since it is adapted to the visual process occurring within the eye of the user by providing an axis which corresponds to the direction of propagation of the light within the eye of the user, generally denoted by the term "optical path". Still, using a different type of coordinate system, such as a Cartesian coordinate system or a polar coordinate system, may also be feasible.

As generally known, each eye of the user comprises a cornea, a crystalline lens, an anterior chamber located between the cornea and the crystalline lens, a retina, a vitreous chamber located between the crystalline lens and the retina, and the fovea, wherein incident light propagates along the axis initially through the cornea, the anterior chamber, the crystalline lens, and the vitreous chamber, thereby focusing the incident light to generate the desired image on the retina, especially on the fovea in order to support the visual process. In an event in which the eye of the user is involved in a fixation of a target, the axis through the eye may, generally, have a point of intersection with the retina at the fovea.

As generally used, the value for the "an axial length" indicates a distance between a first point of intersection of the front curvature of the cornea with the axis and a second point of intersection of the retina with the axis. As further generally used, the value for the "front curvature of the cornea" indicates an amount by which an outward facing surface of the cornea deviates from being a plane. Similarly, the value for the "front back curvature of the cornea" indicates an amount by which an inward facing surface of the cornea deviates from being a plane. Herein, the term "outward" refers to a direction of sight of the eye, while the term "inward" refers to a direction of the propagation of incident light of the eye. As further generally used, the value for the "depth of the cornea" indicates a length of the cornea along the axis. Similarly, the value for the "depth of the anterior chamber", also abbreviated to "ACD" indicates a length of the anterior chamber along the axis between the cornea and the crystalline lens. As further generally used, the value for the "front curvature of the crystalline lens" indicates an amount by which an outward facing surface of the crystalline lens deviates from being a plane. Similarly, the value for the "back curvature of the crystalline lens" indicates an amount by which an inward facing surface of the crystalline lens deviates from being a plane. As further generally used, the value for the "depth of the crystalline lens" indicates a length of the crystalline lens along the axis. As further generally used, the value for the "refractive index" indicates an amount of bending of incident light by a particular substance comprised by a part of the at least one eye of the user, especially the cornea, the crystalline lens, the aqueous humor in the anterior chamber, or a gel-like component as comprised by the vitreous chamber.

In a particular embodiment, the reliability, accuracy and repeatability of the model of the at least one eye of the user may, further, be increased by using at least one additional feature, preferably by using at least one additional feature that is related of the crystalline lens of the at least one eye of the user. In this manner, the model of the eye of the user can, further, be refined, especially to provide corrected values for the ocular aberration having an accuracy below 0.25 dpt, by taking into account that the crystalline lens, in general, exerts a major influence in refracting the incident light within the eye of the user.

In this particular embodiment, the model of the at least one eye of the user may, further, comprise at least one value for at least one of:
- a tilt angle of the crystalline lens;
- a decentration of the crystalline lens;
- a gradient index of the crystalline lens; or
- a retinal curvature of the retina
of the at least one eye of the user. Again, each geometric value, in particular the at least one of the tilt angle or the decentration of the crystalline lens, is provided with respect to the axis on which the reference coordinate system is based.

As generally used, a value for the "tilt angle" indicates a rotation of the crystalline lens around the axis. As further generally used, a value for the "decentration" indicates a deviation of an optical center of the crystalline lens from the axis. As further generally used, a value for the "gradient index" indicates an alteration of the refractive index of a substance comprised by crystalline lens of the eye of the user in a direction perpendicular to the axis. As further generally used, the value for the "retinal curvature" indicates an amount by which a surface of the retina which is configured to, eventually, receive the incident light, deviates from being a plane, wherein, in a particular embodiment, the retinal curvature may, however, vanish by being zero if the retina may be approximated by a plane.

According to step b), the output data is generated, wherein the output data comprises the at least one value for the ocular aberration of the at least one eye of the user. As used herein, the term "output data" refers to a set or a sequence of data that comprises at least one piece of information which is determined in at least one step of a method, especially a computer-implemented method, in particular the method for determining at least one value for an ocular aberration of at least one eye of a user according to the present invention. Herein, the output data may comprise the representative result. For generating the outcome data, input data may be considered, taken into account or evaluated. As further used herein, the term "generating" or any grammatical deviation thereof refers to a process of producing a set or a sequence of data, particularly by using an initial set of data, specifically the input data, and applying a mathematical algorithm, such as a particular functional and/or logical algorithm, or a machine learning model, to it. However, modifying the initial set of data, specifically the input data, in a further fashion may also feasible.

In accordance with the present invention, the produced set of data is the output data, wherein the output data is generated by
- comparing the second information with the third information; and
- adjusting the first information.

As used herein, the term "comparing" or any grammatical deviation thereof refers to a process of producing a result which indicates an amount of a difference between at least one value comprised by the second information and the at least one corresponding value comprised by the third information. By way of example, each the second information and the third information comprises a value for the axial length of the at least one eye of the user, wherein the comparing process indicates the amount by which the two corresponding values may differ from each other.

In a particularly preferred embodiment, the output data may be generated by
- determining at least one differing value between the second information and the third information; and
- adjusting the first information by using the at least one differing value.
Herein, determining the at least one differing value between the second information and the third information may, in particular, comprise
- determining first values for the model of the at least one eye of the user by using the second information;
- determining second values for the model of the at least one eye of the user by using the third information; and
- determining at least one difference between the first values and the second values.

As already indicated above, the third information comprises values for the same pieces of information related to the at least one geometric and optical property of the at least one eye of the user as the second information, wherein, however, both the second information and the third information relate to different point in time. In addition, the first information and the second information may, preferably, be captured in a non-cycloplegic manner without applying cycloplegia to the at least one eye of the user, whereas the third information may have, preferably, been captured under cycloplegia of the at least one eye of the user. As generally known, determining a non-cycloplegic auto-refraction is found to be inaccurate and not suitable for studies of refractive error. Especially, population-based studies on a prevalence of refractive error in school-children have often encountered difficulties in that parents and children refuse to undertake cycloplegic refraction because of blurred vision and photophobia after cycloplegia. However, non-cycloplegic auto-refraction is known to, in general, overestimate myopia and underestimate hyperopia in children whose accommodative responses are active. The present invention, therefore, allows capturing the first information and the second information without cycloplegia of the at least one eye of the user and restricting cycloplegia of the at least one eye of the user to capturing the third information only. In this manner, non-cycloplegic auto-refraction can, especially, be used for population-based studies on refractive errors in children but also for refractive error progression monitoring in myopia progression management in children, both in a reliable, accurate and repeatable fashion. While the third information refers to a previous point in time, the second information refers to a later point in time, wherein both points in time can differ at least one year or at least two years but not more than five years ago from each other. This difference reflects that the corresponding geometric and optical properties of the at least one eye of the user have - under reasonable assumption - remained constant over the period of time as expressed by this difference. If in doubt, the third information can be recorded anew, preferably under cycloplegia of the at least one eye of the user, in order to verify this assumption or to replace the previous values comprised by the third information by renewed values.

As further used herein, the term "adjusting" or any grammatical deviation thereof refers to a process of modifying a value comprised by the first information by a modified value which is based on at least one differing value between the second information and the third information. In this manner, at least one value comprised by the first information may be replaced by a corrected value which is determined by taking into account an influence of the second information on the first information by considering the difference between the second information with respect to the third information. Herein, the corrected value may have an accuracy of less than 0.25 dpt.

In a particularly preferred embodiment, adjusting the first information by using the at least one differing value may comprise
- using the at least one difference between the first values and the second values for determining a modified value for the ocular aberration of the at least one eye of a user at the point in time; and
- using the modified value for the ocular aberration of the at least one eye of the user at the point in time as the output data.
Herein, the at least one difference between the first values and the second values may, especially, comprise a differing value for the at least one refractive index of the crystalline lens of the at least one eye of the as user comprised by the second information compared to the third information.

In a preferred embodiment, the third information which has been determined at a previous point in time which may be one to five years beforehand, can be retrieved from a storage medium. In particular, the storage medium may, be comprised by at least one of a local server, a remote server, or a cloud server. By way of example, the third information may have been determined, especially under cycloplegia of the at least one eye of the user, and, subsequently, be stored by an ophthalmologist in a cloud server together with a reference to the at least one eye of the user, and can be retrieved later by an optometrist or an optician from the cloud server by using the reference to the at least one eye of the user. In this fashion, the third information can be kept on a safe place over a couple of years and can securely be retrieved by using a unique reference number at any time later and from any location all over the world. In addition, further measurements which are configured to determine the first information and the second information can be performed in a non-cycloplegic manner by an optometrist or an optician, who may not be allowed to perform such kinds of measurements under cycloplegia of the at least one eye of the user in all countries, depending on national administrative authorization or approval provisions and requirements.

In a particularly preferred embodiment, the reliability, accuracy and repeatability of the model of the at least one eye of the user can, further, be increased, especially to obtain corrected values for the ocular aberration having an accuracy below 0.25 dpt, by considering that
- the first information refers to a first wavelength; and
- the second information and the third information refer to a second wavelength,
wherein the second wavelength differs from the first wavelength, preferably by at least 100 nm, more preferred of at least 200 nm, more preferred of at least 500 nm.

In this manner, the model can, further, be refined by using
- the first wavelength that may, preferably, assume at least one value within the visible spectral range of 380 nm to 780 nm, such as of 400 nm to 600 nm, especially of 500 nm to 550 nm, to represent a particular wavelength which is used for determining the ocular aberration of the at least one eye of a user, and;
- the second wavelength that may, preferably, assume at least one further value within the near infrared spectral range of 780 nm to 1.5 µm, in particular of 800 nm to 1.2 µm, especially around 1 µm, to represent a further particular wavelength which is, additionally, used for determining the at least one geometric and optical property of the at least one eye of the user.

However, using at least one other value for at least one of the first wavelength range or the second wavelength range may also be feasible. For this purpose, at least one of an automated refractor or a wavefront sensing device configured for capturing the first information may, preferably, be operating at the first wavelength. Similarly, an imaging method operating at the second wavelength may, preferably, be applied for capturing the second information and the third information.

As a result, the first information may comprise a value for a first refractive index of the crystalline lens of the at least one eye of the user as determined for the first wavelength, whereas both the second information and the third information may comprise a value for a second refractive index of the crystalline lens of the at least one eye of the user as determined for the second wavelength. In particular, comparing the second information with the third information as disclosed elsewhere in more detail may, additionally, comprise adjusting the first refractive index determined for the first wavelength with respect to the second refractive index determined for the second wavelength, in particular by modifying the value comprised by the first information by a modified value which is based on the difference in the values between the first wavelength and the second wavelength. Due the difference in the values between the first wavelength and the second wavelength and owing to the generally known course of the refractive index of the crystalline lens of the at least one eye of the user with respect to the wavelength, the value for the second refractive index may, generally, differ from the value for the first refractive index. As a result, this particular embodiment allows further refining the model by using a more appropriate value for the refractive index of the crystalline lens of the at least one eye of the user when determining at least one of the first information, second information and third information. In this manner, the refractive indices of the crystalline lens of the at least one eye of the user as determined for both the first wavelength and the second wavelength are used to modify the eye model in a manner that it may represent the geometric and optical properties of the at least one eye of the user at the first wavelength, at which the at least one value for the first information related to the ocular aberration of the at least one eye of a user may, actually, be determined and, thus, contributes to more reliable, accurate and repeatable results, wherein an accuracy of any corrected value for the ocular aberration may be 0.25 dpt or below.

In a same similar manner, not only the at least one refractive index of the crystalline lens but also of at least one further part of the at least one eye of the user, especially the cornea, the aqueous humor in the anterior chamber, or the vitreous chamber, may be modified.

In a further aspect, the present invention refers to a method for producing at least one spectacle lens for the at least one eye of the user, wherein producing the spectacle lens comprises processing a lens blank, wherein processing the lens blank is based on instructions configured to compensate at least one ocular aberration of the at least one eye of the user, wherein determining the ocular aberration of the at least one eye of the user comprises the steps of the method for determining the at least one value for an ocular aberration of the at least one eye of the user as disclosed elsewhere herein.

In a further aspect, the present invention relates to an apparatus for determining at least one value for an ocular aberration of at least one eye of a user which is, preferably, configured to apply the method for determining at least one value for an ocular aberration of at least one eye of a user as disclosed elsewhere herein. The apparatus comprises at least:
- at least one first measuring device configured to generate first information about an ocular aberration of at least one eye of a user;
- at least one second measuring device configured to generate at least one of second information or third information about at least one geometric and optical property of the at least one eye of the user; and
- a processing device configured to execute the method for determining at least one value for an ocular aberration of at least one eye of a user as disclosed elsewhere herein.

As described above, the apparatus may be a combined apparatus located at a common site which may comprise the at least one first measuring device configured to generate the first information, the at least one second measuring device configured to generate the second information and the third information, and a processing device further configured to control both the at least one first measuring device and the at least one second measuring device. In this manner, it can be ensured that the second information can actually be determined within the same point in time as the first information, in particular in a concurrent fashion or in a consecutive manner.

In a further embodiment, one or more of the devices of the apparatus may be located at individual sites and may comprise at least one communication interface configured to communicate with one or more of the other devices, especially with the processing device. By way of example, the at least one first measuring device and the at least one second measuring device may be a combined apparatus being located at a particular site which may, further, comprise at least one communication interface configured to communicate with one or more processing devices that may be located at one or more different sites via at least one further communication interface comprised by the one or more processing devices.

Alternatively or in addition, one or more of the processing devices may be a virtual device that may be located in a remote site and accessible via internet or a cloud. In this fashion, more complicated algorithms that may be used in executing the method for determining the at least one value for the ocular aberration of the at least one eye of the user could be made available on demand via internet or a cloud. However, further kinds of embodiments of the apparatus may also be feasible.

In a further aspect, the present invention refers to a computer program product which comprises instructions to cause the apparatus for determining at least one value for an ocular aberration of at least one eye of a user to execute at least one of the steps of the method for determining at least one value for an ocular aberration of at least one eye of a user according to the present invention. For this purpose, a computer program may comprise instructions provided by a computer program code which are capable of performing any or all of the steps, in particular steps a) and b), of the method as described elsewhere herein and, thus, to establish determining the at least one value of the ocular aberration of at least one eye of a user when implemented on a computer or a data processing unit. Herein, the computer program code may be provided on an individual storage medium, especially on a non-transient storage medium, such as on a compact disc, on an optical storage medium, directly on a computer or a processing device, in particular comprised by a mobile communication device, or via a network, such as an in-house network or the internet, or in a cloud.

For further details concerning the apparatus and the computer program product for determining at least one value for an ocular aberration of at least one eye of a user as well the method for producing at least one spectacle lens for the at least one eye of the user, reference may be made to the method for determining at least one value for an ocular aberration of at least one eye of a user as disclosed elsewhere herein.

When determining the at least one ocular aberration of at least one eye of a user, the methods, the apparatus, and the computer program product according to the present invention exhibit various advantages with respect to the prior art. In particular, the methods and devices allow determining values for the ocular aberration of one or both eyes of the user with a high reliability, accuracy and repeatability compared to existing methods and devices, such as described in US 2020/0229691 A1. In addition, the methods, the apparatus, and the computer are able to determine values not only for first-order ocular aberrations, such as for the refraction, but also for higher-order ocular aberrations of one or both eyes of the user with a high reliability, accuracy and repeatability, especially with an accuracy of less than 0.25 dpt.

As used herein, the terms "have", "comprise" or "include" or any arbitrary grammatical variations thereof are used in a non-exclusive way. Thus, these terms may refer to both a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

As further used herein, the terms "preferably", "more preferably", "particularly", "more particularly", or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in this way with other features of the invention.

Summarizing, the following Embodiments are particularly preferred within the scope of the present invention:
Embodiment 1. A method for determining at least one value for an ocular aberration of at least one eye of a user, the method comprising the following steps:
   a) providing input data, the input data comprising:
      - first information about an ocular aberration of at least one eye of a user at a point in time;
      - second information about at least one geometric and optical property of the at least one eye of the user at the same point in time; and
      - third information about at least one geometric and optical property of the at least one eye of the user at a previous point in time,
      and
   b) generating output data, the output data comprising the at least one value for the ocular aberration of the at least one eye of the user, wherein the output data is generated by
      - comparing the second information with the third information; and
      - adjusting the first information,
      wherein the second information and the third information is represented in a model of the at least one eye of the user, the model of the at least one eye of the user being based on a value for each of:
      - an axial length;
      - a front curvature, a back curvature, a depth, and at least one refractive index of a cornea;
      - a depth of an anterior chamber and at least one refractive index of an aqueous humor in the anterior chamber;
      - a front curvature, a back curvature, a depth, and at least one refractive index of a crystalline lens; and
      - a depth and at least one refractive index of a vitreous chamber
      of the at least one eye of the user, wherein each geometric value is provided with respect to an axis on which a reference coordinate system is based.
Embodiment 2. The method according to the preceding Embodiment, wherein the model of the at least one eye of the user further comprises at least one value for at least one of:
   - a tilt angle, a decentration, or a gradient index of the crystalline lens; or
   - a retinal curvature of the retina
   of the at least one eye of the user.
Embodiment 3. The method according to any one of the preceding Embodiments, wherein the first information is captured by applying at least one of an automated refractor or a wavefront sensing device to the at least one eye of the user.
Embodiment 4. The method according to any one of the preceding Embodiments, wherein the second information and the third information are captured by applying an imaging method to at least one image of the at least one eye of the user.
Embodiment 5. The method according to any one of the preceding Embodiments, wherein the second information and the third information are captured by applying the same imaging method to the at least one image of the at least one eye of the user.
Embodiment 6. The method according to any one of the preceding Embodiments, wherein the third information is retrieved from a storage medium by using a reference to the at least one eye of the user.
Embodiment 7. The method according to the preceding Embodiment, wherein the storage medium is comprised by at least one of a local server, a remote server, or a cloud server.
Embodiment 8. The method according to any one of the preceding Embodiments, wherein the output data is generated by
   - determining at least one differing value between the second information and the third information; and
   - adjusting the first information by using the at least one differing value.
Embodiment 9. The method according to the preceding Embodiment, wherein determining the at least one differing value between the second information and the third information comprises
   - determining first values for the model of the at least one eye of the user by using the second information;
   - determining second values for the model of the at least one eye of the user by using the third information; and
   - determining at least one difference between the first values and the second values.
Embodiment 10. The method according to the preceding Embodiment, wherein adjusting the first information by using the at least one differing value comprises
   - using the at least one difference between the first values and the second values for determining a modified value for the ocular aberration of the at least one eye of a user at the point in time; and
   - using the modified value for the ocular aberration of the at least one eye of the user at the point in time as the output data.
Embodiment 11. The method according to any one of the two preceding Embodiments, wherein the at least one difference between the first values and the second values comprises a differing value for the at least one refractive index of the crystalline lens of the at least one eye of the user comprised by the second information compared to the third information.
Embodiment 12. The method according to any one of the preceding Embodiments, wherein
   - the first information refers to a first wavelength;
   - the second information and the third information refer to a second wavelength, wherein the second wavelength differs from the first wavelength.
Embodiment 13. The method according to the preceding Embodiment, wherein
   - the first information comprises a value for a first refractive index of at least one of the crystalline lens the cornea, the aqueous humor in the anterior chamber, or the vitreous chamber of the at least one eye of the user determined for the first wavelength;
   - the second information and the third information comprise a value for a second refractive index of at least one of the crystalline lens the cornea, the aqueous humor in the anterior chamber, or the vitreous chamber of the at least one eye of the user determined for the second wavelength.
Embodiment 14. The method according to any one of the two preceding Embodiments, wherein the first information is captured by applying at least one of an automated refractor or a wavefront sensing device operating at the first wavelength to the at least one eye of the user.
Embodiment 15. The method according to any one of the three preceding Embodiments, wherein the second information and the third information are captured by applying an imaging method operating at the second wavelength to at least one image of the at least one eye of the user.
Embodiment 16. The method according to any one of the four preceding Embodiments, wherein comparing the second information with the third information comprises adjusting the first refractive index determined for the first wavelength with respect to the second refractive index determined for the second wavelength.
Embodiment 17. The method according to any one of the preceding Embodiments, wherein the first information comprises at least one of
   - a lower-order ocular aberration; or
   - a higher-order ocular aberration
   of at least one eye of a user.
Embodiment 18. The method according to the preceding Embodiment, wherein the lower-order ocular aberration refers to at least one of
   - a spherical power;
   - a cylindrical power; or
   - a cylinder axis
   of the ocular aberration of the at least one eye of a user.
Embodiment 19. The method according to any one of the two preceding Embodiments, wherein the higher-order ocular aberration refer to at least one third-order aberration of the at least one eye of the user.
Embodiment 20. The method according to any one of the preceding Embodiments,
   - wherein the first information and the second information are captured in a non-cycloplegic manner; and
   - wherein the third information is captured under cycloplegia
   of the at least one eye of the user.
Embodiment 21. The method according to any one of the preceding Embodiments, wherein at least one piece of the output data comprising the at least one value for the ocular aberration of the at least one eye of the user is displayed on a monitor.
Embodiment 22. A method for determining at least one value for an ocular aberration of at least one eye of a user, the method comprising the following steps:
   - capturing third information about at least one geometric and optical property of the at least one eye of the user at a previous point in time under cycloplegia;
   - establishing a model of the at least one eye of the user by using the third information;
   - storing the third information, the model of the at least one eye of the user and a reference to the at least one eye of the user;
   - capturing first information about an ocular aberration of at least one eye of a user at a point in time in a non-cycloplegic manner;
   - capturing second information about at least one geometric and optical property of the at least one eye of the user at the same point in time in a non-cycloplegic manner; and
   - generating the at least one value for the ocular aberration of the at least one eye of the user by
      ∘ comparing the second information with the third information; and
      ∘ adjusting the first information,
   wherein the second information and the third information is represented in a model of the at least one eye of the user, the model of the at least one eye of the user being based on a value for each of:
   - an axial length;
   - a front curvature, a back curvature, a depth, and at least one refractive index of a cornea;
   - a depth of an anterior chamber and at least one refractive index of an aqueous humor in the anterior chamber;
   - a front curvature, a back curvature, a depth, and at least one refractive index of a crystalline lens; and
   - a depth and at least one refractive index of a vitreous chamber
   of the at least one eye of the user, wherein each geometric value is provided with respect to an axis on which a reference coordinate system is based.
Embodiment 23. A method for producing at least one spectacle lens for the at least one eye of the user, wherein producing the spectacle lens comprises processing a lens blank, wherein processing the lens blank is based on instructions configured to compensate at least one ocular aberration of the at least one eye of the user, wherein at least one value for the ocular aberration of the at least one eye is determined by using the method according to any one of Embodiments 1 to 22.
Embodiment 24. An apparatus for determining at least one value for an ocular aberration of at least one eye of a user, the apparatus comprising:
   - at least one first measuring device configured to generate first information about an ocular aberration of at least one eye of a user;
   - at least one second measuring device configured to generate at least one of second information or third information about at least one geometric and optical property of the at least one eye of the user; and
   - a processing device configured to execute a method for determining at least one value for an ocular aberration of at least one eye of the user according to any one of Embodiments 1 to 22.
Embodiment 25. The apparatus according to the preceding Embodiment, wherein the at least one first measuring device is selected from at least one of an auto-refraction measuring device or a wavefront measuring device.
Embodiment 26. The apparatus according to the preceding Embodiment, wherein the at least one first measuring device is configured to operate at a first wavelength.
Embodiment 27. The apparatus according to any one of the preceding apparatus Embodiments, wherein the at least one second measuring device is configured to apply an imaging method to at least one image of the at least one eye of the user.
Embodiment 28. The apparatus according to the preceding Embodiment, wherein the at least one second measuring device is an optical coherence tomograph.
Embodiment 29. The apparatus according to the preceding Embodiment, wherein the at least one second measuring device is configured to operate at a second wavelength.
Embodiment 30. The apparatus according to any one of the preceding apparatus Embodiments, wherein the apparatus further comprises a monitor configured to display at least one piece of output data comprising the at least one value for the ocular aberration of the at least one eye of the user.
Embodiment 31. The apparatus according to any one of the preceding apparatus Embodiments, wherein the processing device is further configured to retrieve the third information from a storage medium by using a reference to the at least one eye of the user.
Embodiment 32. The apparatus according to the preceding Embodiment, wherein the storage medium is comprised by at least one of a local server, a remote server, or a cloud server.
Embodiment 33. The apparatus according to any one of the preceding apparatus Embodiments, wherein the processing device is further configured to compare the second information with the third information by adjusting a first refractive index determined for the first wavelength with respect to a second refractive index determined for the second wavelength.
Embodiment 34. A computer program product comprising instructions to cause the apparatus for determining at least one value for an ocular aberration of at least one eye of a user according to any one of Embodiments 24 to 33 to execute a method for determining at least one value for the ocular aberration of the at least one eye of the user according to any one of Embodiments 1 to 22.
Embodiment 35. The computer program product according to the preceding Embodiment, wherein the instructions cause the processing device comprised by the apparatus for determining the at least one value for the ocular aberration of the at least one eye of the user according to any one of Embodiments 24 to 33 to execute at least one step of the method for determining the at least one value for the ocular aberration of the at least one eye of the user according to any one of Embodiments 1 to 22.

### Short description of the Figures

Further optional features and embodiments of the present invention are disclosed in more detail in the subsequent description of preferred embodiments, preferably in conjunction with the dependent claims. Therein, the respective optional features may be implemented in an isolated fashion as well as in any arbitrary feasible combination, as the skilled person will realize. It is emphasized here that the scope of the invention is not restricted by the preferred embodiments.

In the Figures:
- Figure 1: illustrates a preferred embodiment of an apparatus for determining at least one value of an ocular aberration of at least one eye of a user according to the present invention;
- Figure 2: illustrates a preferred embodiment of a model of the at least one eye of the user as used by the method for determining the at least one value of the ocular aberration of the at least one eye of the user according to the present invention; and
- Figure 3: illustrates preferred embodiments of a method for determining the at least one value of the ocular aberration of the at least one eye of the user according to the present invention.

### Detailed description of the embodiments

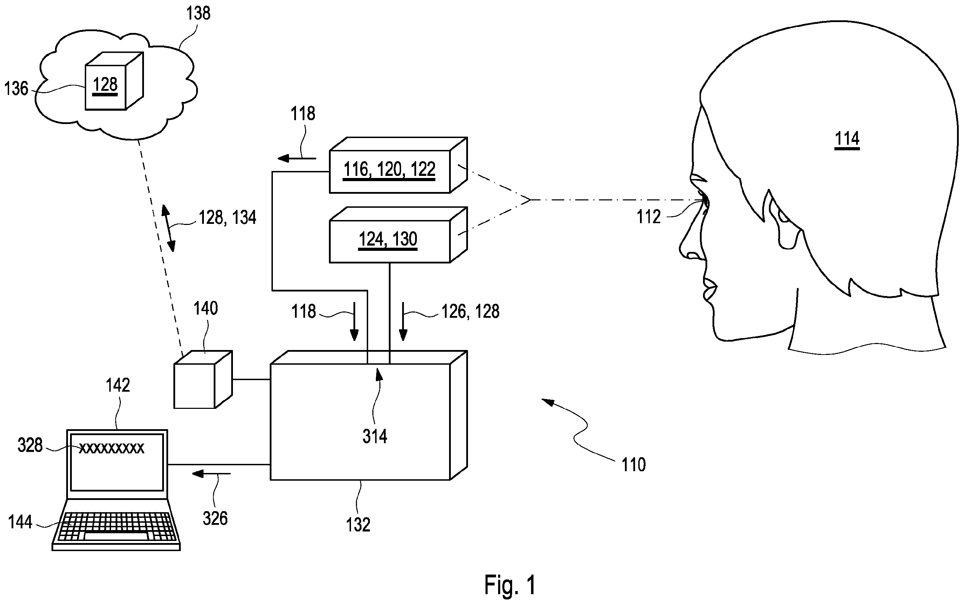

Figure 1 schematically shows a preferred embodiment of an apparatus 110 for determining one or more values for an ocular aberration of one or both eyes 112 of a user 114. As illustrated, the exemplary apparatus 110 of Figure 1 comprises a first measuring device 116, which is configured to generate first information 118 about an ocular aberration of one or both eyes 112 of the user 114. Preferably, the first measuring device 116 may be selected from an auto-refraction measuring device 120 or a wavefront measuring device 122, in particular a Shack Hartmann wavefront sensor; however, a further kind of first measuring device 116 may also be feasible. In particular, the first measuring device 116 may be configured to operate at a first wavelength λ₁.

As further illustrated, the exemplary apparatus 110 of Figure 1, additionally, comprises a second measuring device 124 which is configured to generate second information 126 and/or third information 128 about at least one geometric and optical property of one or both eyes 112 of the user 114. For this purpose, the second measuring device 124 is configured to apply an imaging method to at least one image of one or both eyes 112 of the user 114. Preferably, the second measuring device 124 may be an optical coherence tomograph 130; however, a further kind of second measuring device 124 may also be feasible. In particular, the second measuring device 124 may be configured to operate at a second wavelength λ₂, wherein the second wavelength λ₂ differs from the first wavelength λ₁.

As further illustrated, the exemplary apparatus 110 of Figure 1, additionally, comprises a processing device 132, which is configured to execute a method for determining the one or more values for the ocular aberration of the one or both eyes 112 of a user 114 as elsewhere described herein. In addition, the processing device 132 may, further, be configured to store in or to retrieve third information 128 from a storage medium 136 by using a reference 134 to the respective eye 112 of the user 114. As depicted in Figure 1, the storage medium 136 may, preferably, be comprised by or accessible via a cloud 138. As an alternative (not depicted here), the storage medium 136 may be comprised by a local server or a remote server. For a purpose of communicating with the cloud 138 or, alternatively, with the local or remote server, the apparatus may, further, comprise or have access to a communication interface 140 that is configured for this purpose may, additionally, be present.

Further, the processing device 132 may be configured to control both the first measuring device 116 and the second measuring device 124, especially to cause the apparatus 110 to determine the second information 126 within the same point in time as the first information 118, in particular in a concurrent fashion or a consecutive manner. For a purpose of interaction of the processing device 132 with an examiner, in particular an ophthalmologist, an optometrist, or an optician, a monitor 142 and an input device, such as a keyboard 144, may be comprised or available by the processing device 132. Still further, the processing device 132 may be configured to display the one or more values for the ocular aberration of the one or both eyes 112 of a user 114, in particular on the monitor 142.

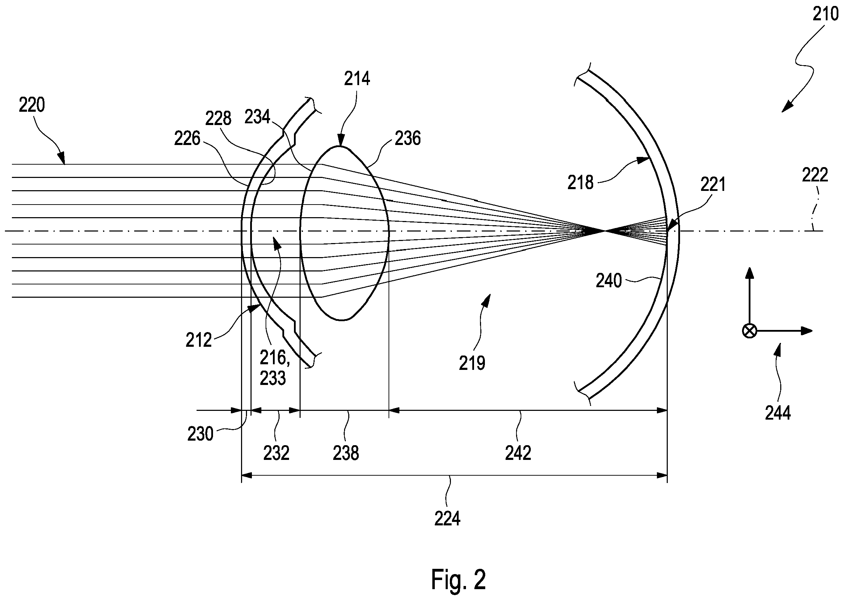

As already indicated above, a model 210 of the eye 112 of the user is applied for determining one or more values for an ocular aberration of one or both eyes 112 of a user 114. Figure 2 schematically shows a preferred embodiment of the model 210. As described above in more detail, the model 210 constitutes simulation configured to provide a geometric and optical representation of the visual process that is occurring within of the eye 112 of the user 114. Herein, the second information 126 comprise values for the same pieces of information which are related to the at least one geometric and optical property of the eye 112 of the user 114 as the third information 128, wherein, however, the third information 128 relates to a previous point in time compared to the second information 126. In addition, the first information 118 and the second information 126 may, preferably, be captured in a non-cycloplegic manner, whereas the third information 128 may have, preferably, been captured previously under cycloplegia of the one or both eyes 112 of the user 114.

As illustrated in Figure 2, each eye 112 of the user 114 comprises
- a cornea 212;
- a crystalline lens 214;
- an anterior chamber 216 located between the cornea 212 and the crystalline lens 214;
- a retina 218;
- a vitreous chamber 219 located between the crystalline lens 214 and the retina 218; and
- a fovea 221,
wherein incident light 220 propagates along an axis 222 initially through the cornea 212 and, subsequently through the anterior chamber 216, the crystalline lens 214, and the vitreous chamber 219, thereby focusing the incident light 220 in a manner to generate an image on the retina 218, especially on the fovea 221 in order to support the visual process. In particular when the eye 112 of the user 114 is involved in a fixation of a target, the axis 222 has a point of intersection with the retina 218 at the fovea 221.

As further shown in Figure 2, the model 210 of the eye 112 of the user 114, especially when the eye 112 of the user 114 is involved in the fixation of a target, is based on a value for each of:
- an axial length 224;
- a front curvature 226, a back curvature 228, a depth 230, and at least one refractive index of the cornea 212;
- a depth 232 of the anterior chamber (ACD) 216 and at least one refractive index of an aqueous humor 233 in the anterior chamber 216;
- a front curvature 234, a back curvature 236, a depth 238, and at least one refractive index of the crystalline lens 214; and
- a depth 242 and at least one refractive index of a gel-like component as comprised by the vitreous chamber 219
of the eye 112 of the user 114. Each geometric value is provided with respect to the axis 222 on which a reference coordinate system 244 is based.

In addition, the model 210 of the eye 112 of the user 114 may, especially when the eye 112 of the user 114 is involved in the fixation of the target, further comprise a value for
- a tilt angle and/or a decentration and/or a gradient index of the crystalline lens 214; and/or
- a retinal curvature 240 of the retina 218
of the eye 112 of the user 114. In addition, using one or more further values for the model 210 of the eye 112 of the user 114 may also be feasible. In this manner, the reliability, accuracy and repeatability of the model 210 of the eye 112 of the user 114 can, further, be increased, especially towards an accuracy of the values for the ocular aberration of one or both eyes 112 of the user 114 of 0.25 dpt or below.

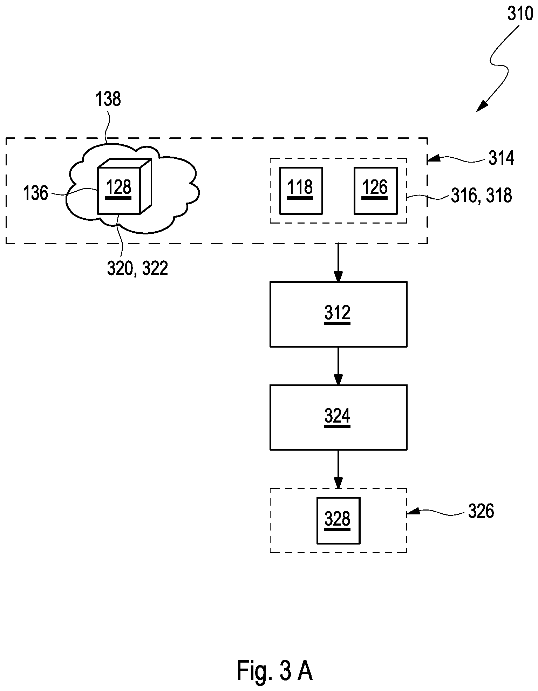

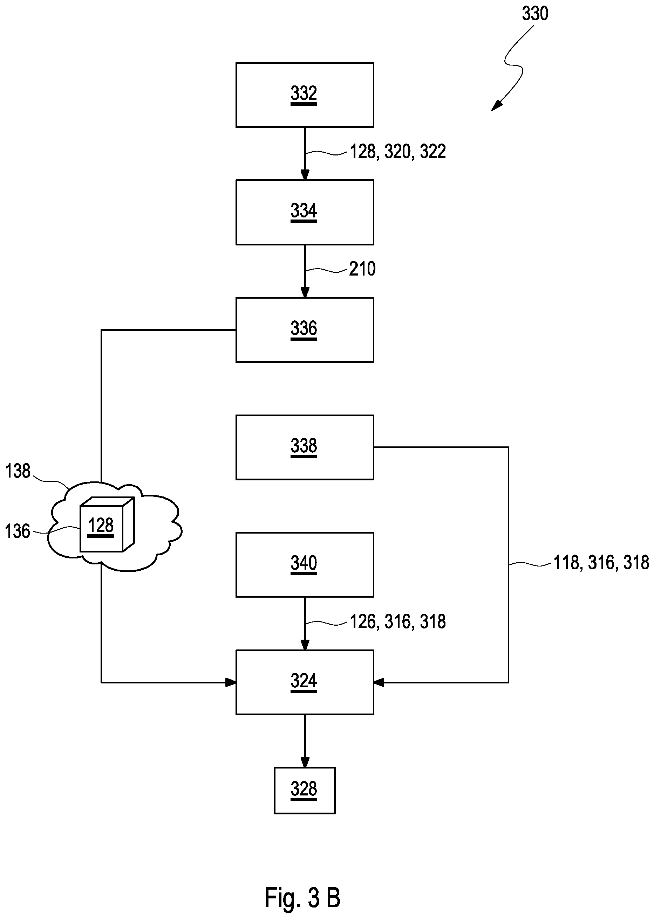

Figure 3 illustrates preferred embodiments of a method for determining the at least one ocular aberration of the one or both eyes 112 of the user 114.

Figure 3A schematically illustrates a first embodiment 310 of the method for determining the at least one value for the ocular aberration of the one or both eyes 112 of the user 114 which has a providing step 312, according to which input data 314 are provided. As indicated above, the input data 314 comprises
- the first information 118 about the ocular aberration of the eye 112 of the user 114 at a point in time 316, preferably determined in a non-cycloplegic manner 318;
- the second information 126 about the at least one geometric and optical property of the eye 112 of the user 114 at the point in time 316, preferably determined in a non-cycloplegic manner 318; and
- the third information 128 about the at least one geometric and optical property of the eye 112 of the user 114 at a previous point in time 320, preferably determined in a under cycloplegia 322.

As further shown in Figure 3A, the first embodiment 310 of the method, further, has a generating step 324 according to which output data 326 are generated. The output data 326 comprise the at least one value 328 for the ocular aberration of the one or both eyes 112 of the user 114. As indicated above, the output data 326 is generated by
- comparing the second information 126 with the third information 128; and
- adjusting the first information 118.

For a purpose of for determining the at least one value for the ocular aberration of the one or both eyes 112 of the user 114 by using the method according to the present invention, the model 210 of the eye 112 of the user 114 as disclosed above in more detail is used for representing the second information 126 and the third information 128.

Figure 3B schematically illustrates a further embodiment 330 of the method for determining the at least one value for the ocular aberration of the one or both eyes 112 of the user 114 which has a first capturing step 332, which comprises capturing the third information 128 about the at least one geometric and optical property of the one or both eyes 112 of the user 114 at the previous point in time 320 under cycloplegia 322.

As further shown in Figure 3B, the further embodiment 330 of the method, further, has an establishing step 334 which comprises generating the model 210 of the one or both eyes 112 of the user 114 by using the third information 118.

Further, the further embodiment 330 of the method as illustrated in Figure 3B has a storing step 336, according to which the third information 118, the parameters of the model 210 of the one or both eyes 112 of the user 114, and the reference 134 to the respective eye 112 of the user 114 is stored, preferably in the storage medium 136, which may, in particular, be comprised by or accessible via a cloud 138. As an alternative, the storage medium 136 may be comprised by a local server or a remote server.

Further, the further embodiment 330 of the method as illustrated in Figure 3B has a second capturing step 338, which comprises capturing the first information 118 about the ocular aberration of the one or both eyes 112 of a user at a point in time 316 in a non-cycloplegic manner 318.

Further, the further embodiment 330 of the method as illustrated in Figure 3B has a third capturing step 340, which comprises capturing the second information 126 about the least one geometric and optical property of the one or both eyes 112 of the user 114 at the same point in time 316 in a non-cycloplegic manner 318;

Further, the further embodiment 330 of the method as illustrated in Figure 3B comprises the generating step 324 according to which the at least one value 328 for the ocular aberration of the one or both eyes 112 of the user 114 is generated by
- comparing the second information 126 with the third information 128; and
- adjusting the first information 118.

For further details with respect to embodiments 310, 330 of the method, reference can be made to the exemplary embodiment of the apparatus 110 of Figure 1.

**List of Reference Signs**

| | | | |
|---|---|---|---|
| 110 | apparatus | 226 | front curvature of cornea |
| 112 | eye | 228 | back curvature of cornea |
| 114 | user | 230 | depth of cornea |
| 116 | first measuring device | 232 | depth of anterior chamber |
| 118 | first information | 233 | aqueous humor |
| 120 | auto-refraction measuring device | 234 | front curvature of crystalline lens |
| 122 | wavefront measuring device | 236 | back curvature of crystalline lens |
| 124 | second measuring device | 238 | depth of crystalline lens |
| 126 | second information | 240 | retinal curvature |
| 128 | third information | 242 | depth of vitreous chamber |
| 130 | optical coherence tomograph | 244 | reference coordinate system |
| 132 | processing device | 310 | first embodiment of method |
| 134 | reference | 312 | providing step |
| 136 | storage medium | 314 | input data |
| 138 | cloud | 316 | point in time |
| 140 | communication interface | 318 | non-cycloplegic manner |
| 142 | monitor | 320 | previous point in time |
| 144 | keyboard | 322 | under cycloplegia |
| 210 | model | 324 | generating step |
| 212 | cornea | 326 | output data |
| 214 | crystalline lens | 328 | value for ocular aberration of eye of user |
| 216 | anterior chamber | | |
| 218 | retina | 330 | further embodiment of method |
| 219 | vitreous chamber | 332 | first capturing step |
| 220 | incident light | 334 | establishing step |
| 221 | fovea | 336 | storing step |
| 222 | axis | 338 | second capturing step |
| 224 | axial length of eye | 340 | third capturing step |

## Claims

1. A method for determining at least one value (328) for an ocular aberration of at least one eye (112) of a user (114), the method comprising the following steps:
a) providing input data (314), the input data (314) comprising:
- first information (118) about an ocular aberration of at least one eye (112) of a user (114) at a point in time (316);
- second information (126) about at least one geometric and optical property of the at least one eye (112) of the user (114) at the same point in time (316); and
- third information (128) about at least one geometric and optical property of the at least one eye (112) of the user (113) at a previous point in time (320),
and
b) generating output data (326), the output data (326) comprising the at least one value (328) for the ocular aberration of the at least one eye (112) of the user (114), wherein the output data (326) is generated by
- comparing the second information (126) with the third information (128); and
- adjusting the first information (118),
**characterized in**
**that** the second information (126) and the third information (128) is represented in a model (210) of the at least one eye (112) of the user (114), the model (210) of the at least one eye (112) of the user (114) being based on a value for each of:
- an axial length (224);
- a front curvature (226), a back curvature (228), a depth (230), and at least one refractive index of a cornea (212);
- a depth (232) of an anterior chamber (216) and at least one refractive index of an aqueous humor (233) in the anterior chamber (216);
- a front curvature (234), a back curvature (236), a depth (238), and at least one refractive index of a crystalline lens (214); and
- a depth (242) and at least one refractive index of a vitreous chamber (219)
of the at least one eye (112) of the user (114), wherein each geometric value is provided with respect to an axis (222) on which a reference coordinate system (244) is based.

2. The method according to the preceding claim, wherein the model (210) of the at least one eye (112) of the user (114) further comprises at least one value for at least one of:
- a tilt angle, a decentration, or a gradient index of the crystalline lens (214); or
- a retinal curvature (240)
of the at least one eye (112) of the user (114).

3. The method according to any one of the preceding claims, wherein
- the first information (118) is captured by applying at least one of an automated refractor (120) or a wavefront sensing device (122) to the at least one eye (112) of the user (114);
- the second information (126) and the third information (128) are captured by applying an imaging method to at least one image of the at least one eye (112) of the user (114); and
- the third information (128) is retrieved from a storage medium (136) by using a reference (134) to the at least one eye (112) of the user (114).

4. The method according to any one of the preceding claims, wherein the output data (326) is generated by
- determining at least one differing value between the second information (126) and the third information (128);
- adjusting the first information (118) by using the at least one differing value.

5. The method according to the preceding claim, wherein determining the at least one differing value between the second information (126) and the third information (128) comprises
- determining first values for the model (210) of the at least one eye (112) of the user (114) by using the second information (126);
- determining second values for the model (210) of the at least one eye (112) of the user (114) by using the third information (128);
- determining at least one difference between the first values and the second values, wherein the at least one difference between the first values and the second values comprises a differing value for the at least one refractive index of the crystalline lens (214) of the at least one eye (112) of the user (114) comprised by the second information (126) compared to the third information (128).
and wherein adjusting the first information (118) by using the at least one differing value comprises
- using the at least one difference between the first values and the second values for determining a modified value for the ocular aberration of the at least one eye (112) of the user (114) at the point in time (316); and
- using the modified value for the ocular aberration of the at least one eye (112) of the user (114) at the point in time (316) as the output data (326).

6. The method according to any one of the preceding claims, wherein
- the first information (118) comprises a value for a first refractive index of at least one of the crystalline lens (214), the cornea (212), the aqueous humor (233) in the anterior chamber (216), or the vitreous chamber (219) of the at least one eye (112) of the user (114) determined for a first wavelength;
- the second information (126) and the third information (128) comprise a value for a second refractive index of at least one of the crystalline lens (214), the cornea (212), the aqueous humor (233) in the anterior chamber (216), or the vitreous chamber (219) of the at least one eye (112) of the user (114) determined for the second wavelength,
wherein the second wavelength differs from the first wavelength.

7. The method according to the preceding claim,
- wherein the first information (118) is captured by applying at least one of an automated refractor (120) or a wavefront sensing device (122) operating at the first wavelength; and
- wherein the second information (126) and the third information (128) are captured by applying an imaging method operating at the second wavelength
to the at least one eye (112) of the user (114).

8. The method according to any one of the two preceding claims, wherein comparing the second information (126) with the third information (130) comprises adjusting the first refractive index determined for the first wavelength with respect to the second refractive index determined for the second wavelength.

9. The method according to any one of the preceding claims,
- wherein the first information (126) and the second information (128) are captured in a non-cycloplegic manner (318); and
- wherein the third information (128) is captured under cycloplegia (322)
of the at least one eye (112) of the user (114).

10. The method according to any one of the preceding claims, wherein at least one piece of the output data (326) comprising the at least one value (328) for the ocular aberration of the at least one eye (112) of the user (114) is displayed on a monitor (142).

11. A method for producing at least one spectacle lens for the at least one eye (112) of the user (114), wherein producing the spectacle lens comprises processing a lens blank, wherein processing the lens blank is based on instructions configured to compensate at least one ocular aberration of the at least one eye (112) of the user (114), wherein at least one value (328) for the ocular aberration of the at least one eye (112) of the user (114) is determined by a method for determining at least one value (328) for an ocular aberration of at least one eye (112) of a user (114) comprising the following steps:
a) providing input data (314), the input data (314) comprising:
- first information (118) about an ocular aberration of at least one eye (112) of a user (114) at a point in time (316);
- second information (126) about at least one geometric and optical property of the at least one eye (112) of the user (114) at the same point in time (316); and
- third information (128) about at least one geometric and optical property of the at least one eye (112) of the user (113) at a previous point in time (320),
and
b) generating output data (326), the output data (326) comprising the at least one value (328) for the ocular aberration of the at least one eye (112) of the user (114), wherein the output data (326) is generated by
- comparing the second information (126) with the third information (128); and
- adjusting the first information (118),
**characterized in**
**that** the second information (126) and the third information (128) is represented in a model (210) of the at least one eye (112) of the user (114), the model (210) of the at least one eye (112) of the user (114) being based on a value for each of:
- an axial length (224);
- a front curvature (226), a back curvature (228), a depth (230), and at least one refractive index of a cornea (212);
- a depth (232) of an anterior chamber (216) and at least one refractive index of an aqueous humor (233) in the anterior chamber (216);
- a front curvature (234), a back curvature (236), a depth (238), and at least one refractive index of a crystalline lens (214); and
- a depth (242) and at least one refractive index of a vitreous chamber (219)
of the at least one eye (112) of the user (114), wherein each geometric value is provided with respect to an axis (222) on which a reference coordinate system (244) is based.

12. An apparatus (110) for determining at least one value (328) for an ocular aberration of at least one eye (112) of a user (114), the apparatus (110) comprising:
- at least one first measuring device (116) configured to generate first information (1189 about an ocular aberration of at least one eye (112) of the user (114);
- at least one second measuring device (124) configured to generate at least one of second information (126) or third information (128) about at least one geometric and optical property of the at least one eye (112) of the user (114); and
- a processing device (132) configured to execute a method for determining at least one value (328) for an ocular aberration of the at least one eye (112) of the user (114) comprising the following steps:
a) providing input data (314), the input data (314) comprising:
- first information (118) about an ocular aberration of at least one eye (112) of a user (114) at a point in time (316);
- second information (126) about at least one geometric and optical property of the at least one eye (112) of the user (114) at the same point in time (316); and
- third information (128) about at least one geometric and optical property of the at least one eye (112) of the user (113) at a previous point in time (320),
and
b) generating output data (326), the output data (326) comprising the at least one value (328) for the ocular aberration of the at least one eye (112) of the user (114), wherein the output data (326) is generated by
- comparing the second information (126) with the third information (128); and
- adjusting the first information (118),
**characterized in**
**that** the second information (126) and the third information (128) is represented in a model (210) of the at least one eye (112) of the user (114), the model (210) of the at least one eye (112) of the user (114) being based on a value for each of:
- an axial length (224);
- a front curvature (226), a back curvature (228), a depth (230), and at least one refractive index of a cornea (212);
- a depth (232) of an anterior chamber (216) and at least one refractive index of an aqueous humor (233) in the anterior chamber (216);
- a front curvature (234), a back curvature (236), a depth (238), and at least one refractive index of a crystalline lens (214); and
- a depth (242) and at least one refractive index of a vitreous chamber (219)
of the at least one eye (112) of the user (114), wherein each geometric value is provided with respect to an axis (222) on which a reference coordinate system (244) is based.

13. The apparatus (110) according to the preceding claim,
- wherein the at least one first measuring device (116) is selected from at least one of an automated refractor (120) or a wavefront measuring device (122);
- wherein at least one second measuring device (1249 is an optical coherence tomograph (130); and
- wherein the processing device (132) is configured to retrieve the third information (128) from a storage medium (136) by using a reference (134) to the at least one eye (112) of the user (114).

14. The apparatus (110) according to the preceding claim,
- wherein the at least one first measuring device (116) is configured to operate at a first wavelength;
- wherein the at least one second measuring device (124) is configured to operate at a second wavelength; and
- wherein the processing device (132) is configured to compare the second information (126) with the third information (128) by adjusting a first refractive index determined for the first wavelength with respect to a second refractive index determined for the second wavelength.

15. A computer program product comprising instructions to the apparatus for determining at least one value (328) for an ocular aberration of at least one eye (112) of a user (114) according to any one of claims 12 to 14 to execute a method for determining at least one value (328) for an ocular aberration of at least one eye (112) of a user (114) comprising the following steps:
a) providing input data (314), the input data (314) comprising:
- first information (118) about an ocular aberration of at least one eye (112) of a user (114) at a point in time (316);
- second information (126) about at least one geometric and optical property of the at least one eye (112) of the user (114) at the same point in time (316); and
- third information (128) about at least one geometric and optical property of the at least one eye (112) of the user (113) at a previous point in time (320),
and
b) generating output data (326), the output data (326) comprising the at least one value (328) for the ocular aberration of the at least one eye (112) of the user (114), wherein the output data (326) is generated by
- comparing the second information (126) with the third information (128); and
- adjusting the first information (118),
**characterized in**
**that** the second information (126) and the third information (128) is represented in a model (210) of the at least one eye (112) of the user (114), the model (210) of the at least one eye (112) of the user (114) being based on a value for each of:
- an axial length (224);
- a front curvature (226), a back curvature (228), a depth (230), and at least one refractive index of a cornea (212);
- a depth (232) of an anterior chamber (216) and at least one refractive index of an aqueous humor (233) in the anterior chamber (216);
- a front curvature (234), a back curvature (236), a depth (238), and at least one refractive index of a crystalline lens (214); and
- a depth (242) and at least one refractive index of a vitreous chamber (219)
of the at least one eye (112) of the user (114), wherein each geometric value is provided with respect to an axis (222) on which a reference coordinate system (244) is based.
